# EUROPÄISCHE PATENTSCHRIFT

(11) **EP 2 539 700 B1**
(45) Veröffentlichungstag und Bekanntmachung des Hinweises auf die Patenterteilung: **12.08.2015**
(21) Anmeldenummer: 10798352.0
(22) Anmeldetag: 28.12.2010
(51) Int. Cl.: G01N 27/04

(54) **VERWENDUNG DER SPEZIFISCHEN WIDERSTANDSMESSUNG ZUR INDIREKTEN BESTIMMUNG DER REINHEIT VON SILANEN UND GERMANEN**
USE OF SPECIFIC RESISTANCE MEASUREMENT TO INDIRECTLY DETERMINE THE PURITY OF SILANES AND GERMANES
UTILISATION D'UNE MESURE DE LA RÉSISTANCE SPÉCIFIQUE POUR LA DÉTERMINATION INDIRECTE DE LA PURETÉ DES SILANES ET DES GERMANES

(30) Priorität: 25.02.2010 DE 102010002342
(43) Veröffentlichungstag der Anmeldung: 02.01.2013
(73) Patentinhaber: Evonik Degussa GmbH, 45128 Essen (DE)
(72) Erfinder: MÜH, Ekkehard, 79618 Rheinfelden (DE); RAULEDER, Hartwig, 79618 Rheinfelden (DE); AMEND, Rainer, 64853 Otzberg (DE); HAJDUK, Martin, 63773 Goldbach (DE)
(86) Internationale Anmeldenummer: PCT/EP2010/070805
(87) Internationale Veröffentlichungsnummer: WO 2011/103941

(56) Entgegenhaltungen:
- DE-A1- 1 523 001
- DE-A1- 2 558 183
- TAYLOR ET AL: "Purification techniques and analytical methods for gaseous and metallic impurities in high-purity silane", JOURNAL OF CRYSTAL GROWTH, ELSEVIER, AMSTERDAM, NL, Bd. 89, Nr. 1, 1. Juni 1988 (1988-06-01), Seiten 28-38, XP024429734, ISSN: 0022-0248, DOI: DOI:10.1016/0022-0248(88)90068-1 [gefunden am 1988-06-01]

## Beschreibung

Die vorliegende Erfindung betrifft ein Verfahren zur indirekten Bestimmung der Reinheit von Silanen und Germanen unter Verwendung eines Gerätes zur Messung des spezifischen Widerstandes. Gattungsgemäße Verfahren sind aus DE 2 558 183 und DE 1 523 001 bekannt.

Die Bestimmung des Gehalts an Verunreinigungen in Prekursor-Verbindungen, die in der Regel Gase sind und die für anspruchsvolle Anwendungen in der Halbleiter- oder Solarindustrie eingesetzt werden, ist schwierig, da die möglichen Nachweis- und Bestimmungsgrenzen von z. B. ICP-MS oder ICP-OES dafür nicht ausreichen. Insbesondere der Gehalt an Verunreinigungen durch Elemente der 3. Hauptgruppe der Elemente des Periodensystems (so genannte p-Typ-Verunreinigungen) und der 5. Hauptgruppe (so genannte n-Typ-Verunreinigungen) ist kritisch für z. B. Halbleiterund Solarzellenprodukte, die Silicium- oder Germaniumschichten aufweisen, die aus den genannten Prekursor-Verbindungen hergestellt werden, z. B. durch Abscheidung im CVD- oder ähnlichen Verfahren.

Ist bei Silicium- oder Germanium-Prekursor-Verbindungen schon eine Qualitätskontrolle bezüglich Verunreinigungen durch Elemente der 3. und 5. Hauptgruppe der Elemente des Periodensystems im eigentlich notwendigen Nachweisbereich bisher schon schwer oder nicht möglich, so ist es nicht verwunderlich, dass bisher im laufenden Betrieb einer industriellen Produktion und/oder Abfüllung von Silanen oder Germanen keine diesbezügliche und standardmäßig durchgeführte Routinequalitätskontrolle erfolgt im Sinne einer mehr oder weniger permanenten Überwachung (Monitoring) der Reinheit der hergestellten und/oder abgefüllten Verbindungen.

Aufgabe der vorliegenden Erfindung war es, ein Verfahren zur Qualitätskontrolle von Silanen und Germanen, insbesondere zur Kontrolle der Reinheit bezüglich der Elemente der 3. und 5. Hauptgruppe des Periodensystems der Elemente, zur Verfügung zu stellen, wobei das Verfahren geeignet sein soll im laufenden Betrieb einer industriellen Produktion und/oder Abfüllung von Silanen oder Germanen die Qualitätskontrolle im Sinne eines zeitgleichen bzw. zeitnahen Monitoring zu ermöglichen. Insbesondere soll das Verfahren geeignet sein, extrem geringe Konzentrationen von Verunreinigungen durch Elemente der 3. und 5. Hauptgruppe des Periodensystems der Elemente zu detektieren und zu quantifizieren, da an Silane und Germane, die zum Beispiel in der Halbleiterindustrie oder der Solarzellenindustrie eingesetzt werden, höchste Reinheitsanforderungen gestellt werden und Kunden aus diesem Bereich einen Qualitätsnachweis insbesondere bezüglich der genannten Elemente der 3. und 5. Hauptgruppe wünschen.

Diese Aufgabe wird erfindungsgemäß gelöst durch ein Verfahren zur industriellen Produktion und/oder Abfüllung von Silanen oder Germanen durch eine indirekte Bestimmung der Reinheit von Silanen und Germanen, wobei aus dem Silan oder German durch Abscheidung aus dem gasförmigen Zustand auf eine Oberfläche eine Silicium-Schicht oder Germanium-Schicht hergestellt wird, dann der spezifische Widerstand der hergestellten Schicht gemessen und aus dem gemessenen Wert anhand zuvor bestimmter Referenzwerte auf die Reinheit des zur Herstellung der Schicht eingesetzten Silans oder Germans geschlossen wird, die Bestimmung der Reinheit der Silane oder Germane im Rahmen einer industriellen Produktion und/oder Abfüllung dieser Silane oder Germane im Sinne einer fortgesetzten Überwachung der Reinheit wiederholt durchgeführt wird und das Verfahren folgende Schritte umfasst:
a) Herstellen oder Bereitstellen des Silans bzw. Germans,
b) Abzweigen oder Entnehmen einer geeigneten Menge des Silans bzw. Germans in regelmäßigen Abständen von den Prozessströmen,
c) Herstellen einer Silicium- bzw. Germanium-Schicht aus zumindest einem Teil der abgezweigten bzw. entnommenen Silan- bzw. Germanmenge durch ein CVD-Verfahren auf einen Silicium-Wafer bzw. Germanium-Wafer,
d) Messen des spezifischen Widerstands an der Oberfläche der hergestellten Schicht, und
e) Zuordnen des gemessenen spezifischen Widerstands zu einem Grad an Reinheit ohne Zuordnung der verunreinigenden Elemente zu einer der Hauptgruppen des Periodensystems der Elemente.

Das Verfahren sieht also vor, dass die Verunreinigungen nicht direkt bestimmt werden durch an den Silanen oder Germanen angewandten Methoden, sondern indirekt durch Messen einer physikalischen Eigenschaft einer Silicium- bzw. Germanium-Schicht, die aus den betreffenden Silanen oder Germanen hergestellt wird, wobei die physikalische Eigenschaft, nämlich der spezifische Widerstand, maßgeblich durch die Konzentration der Verunreinigungen in den eingesetzten Silanen bzw. Germanen, die beim Abscheideprozess in der Schicht gelangen, beeinflusst wird. Insbesondere Verunreinigungen durch Elemente der 3. und der 5. Hauptgruppe des Periodensystems der Elemente beeinflussen den spezifischen Widerstand der Silicium- bzw. Germanium-Schicht.

Der spezifische Widerstand (kurz für spezifischer elektrischer Widerstand oder auch Resistivität) ist eine temperaturabhängige Materialkonstante mit dem Formelzeichen p. Der elektrische Widerstand eines Leiters mit einer über seine Länge konstanten Querschnittsfläche (Schnitt senkrecht zur Längsachse eines Körpers) beträgt: R = ρ A/l, wobei R der elektrische Widerstand, p der spezifische Widerstand, I die Länge und A die Querschnittsfläche des Leiters ist. Folglich kann man p aus der Messung des Widerstands eines Leiterstückes bekannter Geometrie bestimmen.

Im Rahmen der vorliegenden Erfindung erfolgt die Messung des Widerstandes und der Schichtdicke über die so genannte SRP Methode (spreading resistance probe). Dazu wird ein Stück des beschichteten Wafers unter einem definierten Winkel bis auf das Substrat angeschliffen. Die Widerstandsmessung erfolgt dann mittels zweier Probenspitzen, die das gesamte Profil in bestimmten Abständen abtasten und jeweils einen Widerstandswert zu einer bestimmten Schichtdicke ergeben. Über den Anschliffwinkel und die Weglänge lässt sich auch die Schichtdicke berechnen. Die Methode ist in mehreren Normen detailliert beschrieben und legt das zuvor beschriebene Vorgehen fest. Die hier verwendeten Methoden folgen den SEMI-Normen MF672 sowie MF674 auf die in der MF672 verwiesen wird. Die SEMI-Norm MF672 ist eine Erweiterung der SEMI-Norm MF525. Die SEMI-Normen sind auch als ASTM-Normen (z. B. ASTM F 672-80) veröffentlicht.

Da das erfindungsgemäße Verfahren insbesondere für den industriellen Bereich von großem Nutzen sein kann, sind bevorzugte Silane und Germane solche, die in industriellem Maßstab eingesetzt werden. Vorzugsweise sind daher die Silane und Germane ausgewählt aus den unsubstituierten Mono-, Di- oder Trisilanen oder den unsubstituierten Mono-, Di- oder Trigermanen. Besonders bevorzugt sind dabei SiH₄, Si₂H₆, Si₃H₈ undGeH₄.

Wie bereits erwähnt, beeinflussen insbesondere Verunreinigungen durch Elemente der 3. und der 5. Hauptgruppe des Periodensystems der Elemente den spezifischen Widerstand der ansonsten höchstreinen Silicium- bzw. Germanium-Schicht. Vorzugsweise bezieht sich der Begriff "Reinheit" daher auf die - möglichst geringe - Konzentration von Elementen aus der 3. und 5. Hauptgruppe des Periodensystems der Elemente in den gemessenen Schichten und somit auch in den zur Herstellung dieser Schichten eingesetzten Silanen bzw. Germanen. Verunreinigungen durch Elemente aus der 3. Hauptgruppe des Periodensystems der Elemente bezeichnet man dabei als p-Typ-Verunreinigungen. Verunreinigungen durch Elemente aus der 5. Hauptgruppe hingegen als n-Typ-Verunreinigungen.

Die indirekte Bestimmung der Reinheit der untersuchten Silane oder Germane kann dabei im einfachsten Fall eher qualitativ erfolgen in dem Sinne, dass anhand des gemessenen spezifischen Widerstand eine Aussage derart möglich ist, dass das eingesetzte Silan oder German ein zuvor festgelegtes Mindestmaß an "Reinheit" aufweist, also relativ zu einem Referenzsilan oder -german reiner oder unreiner an den spezifischen Widerstand beeinflussenden Verunreinigungen ist.

Das erfindungsgemäße Verfahren ermöglicht es darüber hinaus auch, durch entsprechend sorgfältige Erstellung von Referenzwerten und Referenzkurven, die weiter unter näher beschrieben werden, zu einer relativ genauen quantitativen Bestimmung der Reinheit des eingesetzten Silans bzw. Germans zu gelangen.

In einer Ausführungsform des erfindungsgemäßen Verfahrens kann auf den Gesamtgehalt an Elementen der 3. und der 5. Hauptgruppe des Periodensystems der Elemente geschlossen werden, wobei diese Elemente nicht der jeweiligen Hauptgruppe zugeordnet werden.

In dem erfindungsgemäßen Verfahren erfolgt die Abscheidung aus dem gasförmigen Zustand auf die Oberfläche vorzugsweise durch ein CVD-Verfahren im Falle der Bestimmung der Reinheit von Silanen auf einen Silicium-Wafer und im Falle der Bestimmung der Reinheit von Germanen auf einen Germanium-Wafer.

Bei den CVD-Verfahren werden in geeigneten Reaktoren (z. B. Applied Centura HAT, ASM Epsilon 2000 oder Novellus Concept One 200) Prekursoren auf Silicium- bzw. Germaniumbasis oder Mischungen von Prekursoren verdampft und an heißen Oberflächen (z. B. einem Siliciumwafer) zum festen Schichtmaterial abgeschieden. Vorteilhaft haben sich auch neuere Abwandlungen dieses Verfahrens, wie z. B. RPCVD (reduced pressure chemical vapor deposition), LPCVD (low pressure chemical vapor deposition) und PECVD (plasma enhanced pressure chemical vapor deposition) erwiesen, die schnellere Abscheidung bei zum Teil deutlich reduzierter Temperatur ermöglichen (Literatur: Andreas Weber, "Chemical vapor deposition-Eine Übersicht", Spektrum der Wissenschaft, April 1996, 86 - 90).

Als Substrate kommen kommerziell erhältliche Si- bzw. Ge-Wafer in Betracht. Durchmesser: 1 - 12 inch, Czochralski oder Float-zone Typ, p- oder n-vordotiert, Orientierung <100> oder <111> muss in der Kalibrierung abgebildet sein, spezifischer Widerstand 0,001 - 15000 Ωcm. Es können ein- oder zweiseitig polierte Wafer zum Einsatz kommen. Die Wafer werden im Normalfall im Epitaxiereaktor vor der Beschichtung für kurze Zeit i.d.R. 0,5 - 5 min im H₂-Strom aufgeheizt, um die natürliche Oxidschicht zu entfernen. Anschließend erfolgt dann der Abscheideschritt, ohne dass der Wafer aus der Kammer entfernt wird.

Zur Bestimmung des Endwertes des spezifischen Widerstand, der sich ab einer gewissen Schichtdicke einstellt, sollte die Silicium-Schicht oder die Germanium-Schicht im Falle eines dotierten Wafers mit einem spezifischen Widerstandswert von < 1000 Ωcm eine Dicke von 5 bis 100 µm, bevorzugt 8 bis 50 µm, besonders bevorzugt 10 bis 20 µm, aufweisen und im Falle eines schwach dotierten Wafers mit einem spezifischen Widerstandswert von > 1000 Ωcm (z. B. bei Flow-Zone-Wafern) eine Dicke von 1 bis 50 µm, bevorzugt 2 bis 25 µm, besonders bevorzugt 3 bis 10 µm. Diese Mindestdicke garantiert, dass sich der gemessene spezifische Widerstand bei weiter anwachsender Schicht in der Regel nicht mehr signifikant ändert, sondern dann ausschließlich durch das abzuscheidende Gas beeinflusst wird. Die Dicken werden mit Hilfe des in den SEMI-Normen beschriebenen Messgerätes ermittelt.

Das erfindungsgemäße Verfahren ist so ausgestaltet, dass die Bestimmung der Reinheit der Silane oder Germane im Sinne einer fortgesetzten Überwachung der Reinheit wiederholt durchgeführt wird, entsprechend einem zeitgleichen oder zeitnahen Monitoring, im Rahmen einer industriellen Produktion und/oder industriellen Abfüllung dieser Silane oder Germane. Dabei werden geeignete Mengen des Silans bzw. Germans in regelmäßigen Abständen von den normalen Prozessströmen abgezweigt oder entnommen und der Epitaxie und anschließenden Messung zugeführt. Die Vorteile gegenüber einer Beprobung von zuvor abgefüllten Flaschen sind: kein Flaschen- oder Gebindewechsel bei jeder neuen Charge, keine Kontaminationsmöglichkeiten beim Flaschenwechsel, sowie eine kontinuierliche Prozesskontrolle.

Wird das erfindungsgemäße Verfahren durchgeführt, umfasst es mindestens folgende Schritte:
a) Herstellen oder Bereitstellen des Silans bzw. Germans,
b) Abzweigen oder Entnehmen einer geeigneten Menge des Silans bzw. Germans in regelmäßigen Abständen von den Prozessströmen,
c) Herstellen einer Silicium- bzw. Germanium-Schicht aus zumindest einem Teil der abgezweigten bzw. entnommenen Silan- bzw. Germanmenge durch ein CVD-Verfahren auf einen Silicium-Wafer bzw. Germanium-Wafer,
d) Messen des spezifischen Widerstands an der Oberfläche der hergestellten Schicht, und
e) Zuordnen des gemessenen spezifischen Widerstands zu einem Grad an Reinheit ohne Zuordnung der verunreinigenden Elemente zu einer der Hauptgruppen des Periodensystems der Elemente.

Das Abzweigen oder Entnehmen der geeigneten Menge des gasförmigen oder flüssigen Silans bzw. Germans kann dabei über geeignete Rohrleitungen z. B. aus einem Tank erfolgen. Das Silan bzw. German kann dann gegebenenfalls über einen Verdampfer in den Epitaxiereaktor überführt werden.

Das Messen des spezifischen Widerstands "an der Oberfläche" kann ohne oder mit Vorbehandlung (z. B. durch Reinigen oder Polieren) direkt auf der Oberfläche erfolgen oder aber z. B. nach Anschleifen nahe unterhalb der ursprünglichen Oberfläche.

Wird nach dieser Variante des Verfahrens nur ein einziger Widerstandswert bestimmt, kann nur auf einen Gesamtgehalt an Elementen der 3. und 5. Hauptgruppe des Periodensystems der Elemente geschlossen werden ohne Zuordnung welche Elemente Ursache der Verunreinigung sind.

Die unter Schritt d) durchzuführende Messung erfolgt dabei vorzugsweise den SEMI-Normen MF672 sowie MF674.

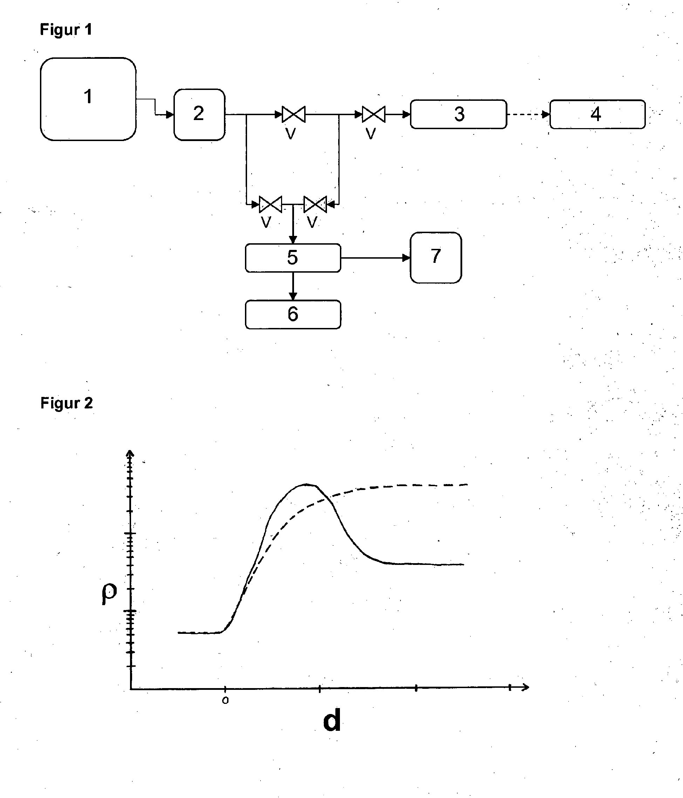

Im Schritt e) wird der jeweils gemessene spezifische Widerstandswert dann bevorzugt in die Ladungsträgerkonzentration umgerechnet. Im Falle dass ein Widerstandsprofil ausgewertet wird, wird der Widerstandsendwert genommen, also der Wert, der sich bei steigender Schichtdicke nicht mehr ändert sondern das abschließende Plateau bildet (vgl. Figur 2). Die Umrechnungen hierzu können Thurber, Mattis, Liu und Filiben folgen (in: National Bureau of Standards Special Publication 400-64, The Relationship between Resistivity and Dopant density for Phosphorous and Boron Doped Silicon, May 1981). Aus der Ladungsträgerkonzentration (Volumenanteil) können dann über die Dichte des Materials (näherungsweise der Wert für Si) die gewichtsanteiligen Werte z. B. in ppb oder ppt errechnet werden. Vorliegend wird das Widerstandsmessgerätes SSM 2000 (der Firma Semilab) mit der entsprechend mitgelieferten Auswertesoftware (analysis.exe) verwendet. Die Auswertesoftware ist so programmiert, dass direkt die Ladungsträgerkonzentration angegeben wird.

Das hier beschriebene Verfahren sieht also im Kern vor, dass zur indirekten Bestimmung der Reinheit von Silanen und Germanen die spezifische Widerstandsmessung verwendet wird. Im typischen Fall umfasst dabei die erfindungsgemäße Verwendung bei der indirekten Bestimmung der Reinheit der Silane bzw. Germane das Herstellen einer Silicium- bzw. Germanium-Schicht aus zumindest einem Teil des Silans bzw. Germans und das gleichzeitige oder anschließende Messen des spezifischen Widerstandes dieser Silicium- bzw. Germanium-Schicht.
Figur 1 zeigt schematisch eine Produktions- und Abfüllanlage für Silane oder Germane einschließlich einer Station für Qualitätskontrolle umfassend ein Gerät für die Messung des spezifischen Widerstandes.
Figur 2 zeigt schematisch jeweils einen typischen Kurvenverlauf für die Abhängigkeit des spezifischen Widerstandes von der Schichtdicke für eine p-Typ- und eine n-Typ-Verunreinigung.

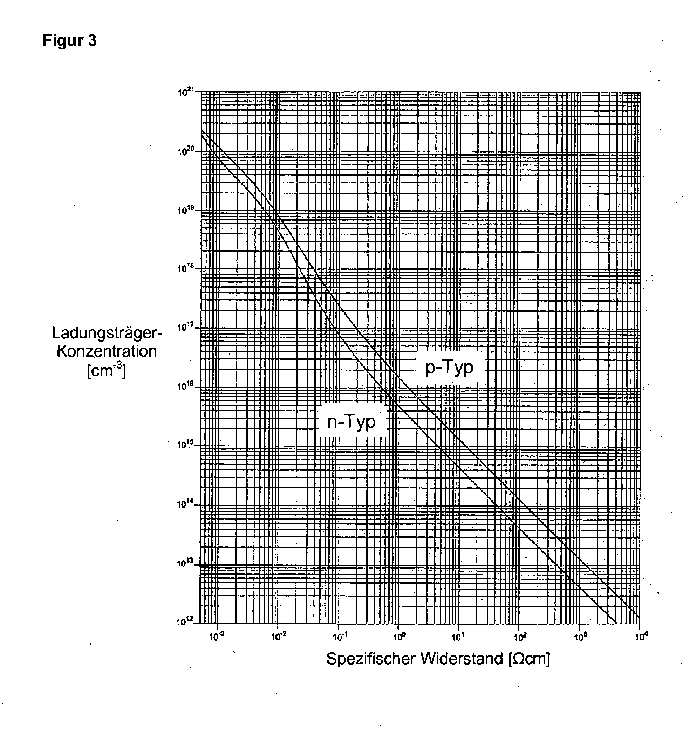

Figur 3 zeigt den logarithmischen Auftrag der Ladungsträgerkonzentration in cm⁻³ (Carrier Concentration) in Abhängigkeit des spezifischen Widerstandes in Ωcm (Resistivity) für p-Typ-Verunreinigungen (z. B. Bor) und für n-Typ-Verunreinigungen (z. B. Phosphor).

In den in Figur 2 gezeigten Kurvenverläufen sind typische Kurvenverläufe für den spezifischen Widerstand p gegen die Schichtdicke d aufgetragen. Auf der linken Seite < 0 ist konstant der spezifische Widerstand des Substratwafers zu erkennen. Mit zunehmender Epitaxieschichtdicke steigt der Widerstand an und strebt im Falle der gestrichelten Linie einem Grenzwert/Plateau entgegen. Dies ist der Fall, wenn Waferdotierung und vorwiegende Verunreinigung in der abgeschiedenen Siliciumoder Germanium-Schicht gleichen Typs sind, also n-Typ oder p-Typ. Unterscheiden sich Waferdotierung und vorwiegende Verunreinigung in der abgeschiedenen Schicht, also n-Wafer/p-Schicht oder p-Wafer/n-Schicht, erhält man den durchgezogenen Kurvenverlauf mit einem Maximum, an dem Dotierung aus dem Wafer und Verunreinigung in der Schicht sich gerade aufheben. Auch hier wird dann ein Plateau erreicht, welches nur durch die Verunreinigung(en) aus der Silicium- oder Germanium-Schicht bestimmt wird. Zu bemerken ist, dass die Änderung des spezifischen Widerstandes beim Übergang vom Wafer zur Schicht nicht sprungartig erfolgt, da die dotierenden bzw. verunreinigenden Elemente über die Grenzschicht hinweg auf die andere Seite einwandern oder diffundieren. Es ist bei Kenntnis der Waferdotierung also möglich zu erkennen, ob es sich um eine p- oder n-Typ-Verunreinigung handelt.

### Beispiel 1

Hochreines SiCl₄ wurde aus einem Vorratstank über eine Leitung in einen Verdampfer überführt, der wiederum an den Gaseinlass eines ASM 2000 Epitaxiereaktors angeschlossen war. Das gasförmige SiCl₄ wurde in Gegenwart von Wasserstoff (Partialdrücke H₂ 1 bar, SiCl₄ 10⁻³ bar) bei 1150 °C auf einem 100 mm Siliciumwafer (p-type, ca. 30 Ωcm) bis zu einer Schichtdicke von 23 µm abgeschieden. Der beschichtete Wafer wurde gemäß SEMI MF 674 vorbereitet und dann gemäß SEMI MF 525/672 auf einem SRP Messgerät (SSM 2000) vermessen. Der Kurvenverlauf wies kein Maximum auf. Der spezifische Widerstand im Bereich des Plateaus (vgl. Abb. 2 gestrichelte Kurve) lag bei > 100 Ωcm. Der Kurvenverlauf lässt auf eine p-Typ-Verunreinigung wie Bor schließen. Die Ladungsträgerkonzentration wurde zu < 1,5 x 10¹⁴ cm⁻³ ermittelt.

### Beispiel 2

"Electronic grade" Dichlorsilan wurde aus einem Vorratstank über eine Leitung an den Gaseinlass eines ASM 2000 Epitaxiereaktors herangeführt. Das gasförmige Dichlorsilan wurde in Gegenwart von Wasserstoff (Partialdrücke H₂ 1 bar, SiH₂Cl₂ 10^{- 3} bar) bei 950 °C auf einem 100 mm Siliciumwafer (p-type, ca. 30 Ωcm) bis zu einer Schichtdicke von 16 µm abgeschieden. Der beschichtete Wafer wurde gemäß SEMI MF 674 vorbereitet und dann gemäß SEMI MF 525/672 auf einem SRP Messgerät (SSM 2000) vermessen. Der Kurvenverlauf wies ein Maximum auf. Der spezifische Widerstand im Bereich des Plateaus (vgl. Abb. 2 durchgezogene Kurve) lag bei > 400 Ωcm. Der Kurvenverlauf lässt auf eine n-Typ-Verunreinigung wie Phosphor oder Arsen schließen. Die Ladungsträgerkonzentration wurde zu < 1,1 x 10¹³ cm⁻³ ermittelt.

### Beispiel 3

Hochreines Monosilan wurde aus einem Vorratstank über eine Leitung an den Gaseinlass eines ASM 2000 Epitaxiereaktors herangeführt. Das gasförmige Monosilan wurde in Gegenwart von Wasserstoff (Partialdrücke H₂ 1 bar, SiH₄ 10⁻³ bar) bei 950 °C auf einem 100 mm Siliciumwafer (p-type, ca. 30 Ωcm) bis zu einer Schichtdicke von 15 µm abgeschieden. Der beschichtete Wafer wurde gemäß SEMI MF 674 vorbereitet und dann gemäß SEMI MF 525/672 auf einem SRP Messgerät (SSM 2000) vermessen. Der Kurvenverlauf wies kein Maximum auf. Der spezifische Widerstand im Bereich des Plateaus (vgl. Abb. 2 gestrichelte Kurve) lag bei > 800 Ωcm. Der Kurvenverlauf lässt auf eine p-Typ-Verunreinigung wie Bor schließen. Die Ladungsträgerkonzentration wurde zu < 1,7 x 10¹³ cm⁻³ ermittelt.

### Bezugszeichenliste

- (1): Produktion und Aufreinigung
- (2): Vorratstank
- (3): Abfüllung
- (4): Versand
- (5): Abscheidung (Epitaxie)
- (6): spezifische Widerstandsmessung
- (7): Verbrennungsanlage

## Patentansprüche

1. Verfahren zur industriellen Produktion und/oder Abfüllung von Silanen oder Germanen durch eine indirekte Bestimmung der Reinheit von Silanen und Germanen,
wobei aus dem Silan oder German durch Abscheidung aus dem gasförmigen Zustand auf eine Oberfläche eine Silicium-Schicht oder Germanium-Schicht hergestellt wird, dann der spezifische Widerstand der hergestellten Schicht gemessen und aus dem gemessenen Wert anhand zuvor bestimmter Referenzwerte auf die Reinheit des zur Herstellung der Schicht eingesetzten Silans oder Germans geschlossen wird, die Bestimmung der Reinheit der Silane oder Germane im Rahmen einer industriellen Produktion und/oder Abfüllung dieser Silane oder Germane im Sinne einer fortgesetzten Überwachung der Reinheit wiederholt durchgeführt wird und
das Verfahren folgende Schritte umfasst:
a) Herstellen oder Bereitstellen des Silans bzw. Germans,
b) Abzweigen oder Entnehmen einer geeigneten Menge des Silans bzw. Germans in regelmäßigen Abständen von den Prozessströmen,
c) Herstellen einer Silicium- bzw. Germanium-Schicht aus zumindest einem Teil der abgezweigten bzw. entnommenen Silan- bzw. Germanmenge durch ein CVD-Verfahren auf einen Silicium-Wafer bzw. Germanium-Wafer,
d) Messen des spezifischen Widerstands an der Oberfläche der hergestellten Schicht, und
e) Zuordnen des gemessenen spezifischen Widerstands zu einem Grad an Reinheit ohne Zuordnung der verunreinigenden Elemente zu einer der Hauptgruppen des Periodensystems der Elemente.

2. Verfahren nach Anspruch 1, wobei die Silane oder Germane ausgewählt sind aus den unsubstituierten Mono-, Di- oder Trisilanen oder den unsubstituierten Mono-, Di- oder Trigermanen, wobei die Silane oder Germane bevorzugt ausgewählt sind aus der Gruppe, die SiH₄, Si₂H₆, Si₃H₈, und GeH₄ umfasst.

3. Verfahren nach einem der vorherigen Ansprüche, wobei sich die Reinheit auf den Gesamtgehalt an Elementen der 3. und der 5. Hauptgruppe des Periodensystems der Elemente bezieht unabhängig von der Zuordnung zur jeweiligen Hauptgruppe.

4. Verfahren nach einem der vorherigen Ansprüche, wobei die Abscheidung aus dem gasförmigen Zustand auf die Oberfläche durch ein CVD-Verfahren im Falle der Bestimmung der Reinheit von Silanen auf einen Silicium-Wafer und im Falle der Bestimmung der Reinheit von Germanen auf einen Germanium-Wafer erfolgt.

5. Verfahren nach einem der vorherigen Ansprüche, wobei die Silicium-Schicht oder die Germanium-Schicht im Falle eines dotierten Wafers mit einem spezifischen Widerstandswert von < 1000 Ωcm eine Dicke von 5 bis 100 µm und im Falle eines schwach dotierten Wafers mit einem spezifischen Widerstandswert von > 1000 Ωcm eine Dicke von 1 bis 50 µm aufweist.

## Claims

1. Process for industrial production and/or dispensing of silanes or germanes by indirectly determining the purity of silanes and germanes, wherein a silicon layer or germanium layer is produced on a surface from the silane or germane by deposition from the gaseous state, then the specific resistivity of the layer produced is measured and the value measured, using reference values determined beforehand, is used to conclude the purity of the silane or germane used to produce the layer, the determination of the purity of the silanes or germanes is performed repeatedly in the course of industrial production and/or dispensing of these silanes or germanes for the purposes of continuous monitoring of purity and the process comprises the following steps:
a) preparing or providing the silane or germane,
b) branching off or withdrawing a suitable amount of the silane or germane at regular intervals from the process streams,
c) producing a silicon or germanium layer from at least a portion of the branched-off or withdrawn amount of silane or germane by a CVD process on a silicon wafer or germanium wafer,
d) measuring the specific resistivity on the surface of the layer produced, and
e) attributing the specific resistivity measured to a degree of purity without attributing the contaminating elements to one of the main groups of the periodic table of the elements.

2. Process according to Claim 1, wherein the silanes or germanes are selected from the unsubstituted mono-, di- or trisilanes or the unsubstituted mono-, di- or trigermanes, the silanes or germanes preferably being selected from the group comprising SiH₄, Si₂H₆, Si₃H₈, and GeH₄.

3. Process according to either of the preceding claims, wherein the purity relates to the total content of elements of main groups 3 and 5 of the periodic table of the elements, irrespective of the attribution to either main group.

4. Process according to any one of the preceding claims, wherein the deposition from the gaseous state onto the surface is effected by a CVD process onto a silicon wafer in the case of determination of the purity of silanes, and onto a germanium wafer in the case of determination of the purity of germanes.

5. Process according to any one of the preceding claims, wherein the silicon layer or the germanium layer in the case of a doped wafer having a specific resistivity of < 1000 Ωcm has a thickness of 5 to 100 µm, and in the case of a lightly doped wafer having a specific resistivity of > 1000 Ωcm has a thickness of 1 to 50 µm.

## Revendications

1. Procédé pour la production et/ou le soutirage industriel(le)(s) de silanes ou de germanes par détermination indirecte de la pureté de silanes et de germanes,
une couche de silicium ou une couche de germanium étant produite sur une surface à partir du silane ou du germane par dépôt à partir de l'état gazeux, la résistance spécifique de la couche produite étant ensuite mesurée et une conclusion relative à la pureté du silane ou du germane utilisé pour la production de la couche étant tirée à partir de la valeur mesurée à l'aide d'une valeur de référence déterminée au préalable, la détermination de la pureté des silanes ou des germanes étant réalisée de manière répétée dans le sens d'une surveillance continue dans le cadre d'une production et/ou d'un soutirage industriel(le)(s) de ces silanes ou germanes et le procédé comprenant les étapes suivantes :
a) préparation ou mise à disposition du silane ou du germane,
b) déviation ou prélèvement d'une quantité appropriée du silane ou du germane des flux de procédé à des intervalles réguliers,
c) production d'une couche de silicium ou de germanium à partir d'au moins une partie de la quantité de silane ou de germane déviée ou prélevée par un procédé CVD sur une tranche de silicium ou une tranche de germanium,
d) mesure de la résistance spécifique à la surface de la couche produite et
e) attribution de la résistance spécifique mesurée à un degré de pureté sans attribution des éléments contaminants à un des groupes principaux du système périodique des éléments.

2. Procédé selon la revendication 1, les silanes ou les germanes étant choisis parmi les monosilanes, les disilanes ou les trisilanes non substitués ou les monogermanes, les digermanes ou les trigermanes non substitués, les silanes ou les germanes étant de préférence choisis dans le groupe comprenant SiH₄, Si₂H₆, Si₃H₈ et GeH₄.

3. Procédé selon l'une quelconque des revendications précédentes, la pureté se rapportant à la teneur totale en éléments du 3ème et du 5ème groupe principal du système périodique des éléments, indépendamment de l'attribution au groupe principal respectif.

4. Procédé selon l'une quelconque des revendications précédentes, le dépôt à partir de l'état gazeux sur la surface par un procédé CVD ayant lieu, dans le cas de la détermination de la pureté de silanes, sur une tranche de silicium et, dans le cas de la détermination de la pureté de germanes, sur une tranche de germanium.

5. Procédé selon l'une quelconque des revendications précédentes, la couche de silicium ou la couche de germanium, dans le cas d'une tranche dopée d'une résistance spécifique < 1000 Ωcm, présentant une épaisseur de 5 à 100 µm et, dans le cas d'une tranche faiblement dopée d'une résistance spécifique > 1000 Ωcm, présentant une épaisseur de 1 à 50 µm.
